# EUROPEAN PATENT APPLICATION

(11) **EP 3 348 642 A1**
(43) Date of publication of application: **18.07.2018**
(21) Application number: 16838600.1
(22) Date of filing: 26.08.2016
(51) Int. Cl.: C12N 15/11, C12Q 1/68

(54) **MOLECULAR WEIGHT INTERNAL STANDARD AND APPLICATIONS THEREOF**

(30) Priority: 27.08.2015 CN 201510535029
(71) Applicant: Nuhigh Biotechnologies Co., Ltd, Suzhou, Jiangsu 215123 (CN)
(72) Inventor: BI, Wanli, Suzhou Jiangsu 215123 (CN); LU, Weihua, Suzhou Jiangsu 215123 (CN)
(74) Representative: V.O.
(86) International application number: PCT/CN2016/096938
(87) International publication number: WO 2017/032342

(57) **Abstract**

Provided is a molecular weight internal standard, consisting of n nucleotide fragments that have different molecular weights and identifiable markers, the n nucleotide fragments comprising at least p nucleotide fragment pairs differing by one base, and one fragment of the nucleotide fragment pair differing by one base having m bases, and the other fragment having m+1 or m-1 bases; p is 1 to n/2 when n is an even number and p is 1 to (n-1)/2 when n is an odd number. Compared with current commercial molecular weight internal standards, the molecular weight internal standard can be used to identify a single base and differentiate PCR product fragments differing by one base, and is applicable to STR analysis, sequencing, large fragment analysis, and genome analysis.

## Description

### CROSS REFERENCE TO RELATED APPLICATIONS

This application claims the priority of Chinese Patent Application No. 201510535029.4, filed on August 27th, 2015, and titled with "MOLECULAR WEIGHT INTERNAL STANDARD AND APPLICATIONS THEREOF", and the disclosures of which are hereby incorporated by reference.

### FIELD

The present invention relates to the field of biotechnology, specifically to a molecular weight standard and application thereof, and in particular, to a molecular weight standard that can clearly identify single base difference and ensure correct genotyping, and application thereof.

### BACKGROUND

Genome scanning is a method which is used to find markers which are tightly linked to specific traits or genes from the genome-wide genetic markers, and locate the relevant genes on chromosomes. Genotyping, which is also known as genotypic assay, is a technology for determining individual genotype by biological detection method, including Polymerase Chain Reaction (PCR), DNA fragment analysis, Allele Specific Oligonucleotide probes (ASO probes), gene sequencing, nucleic acid hybridization, gene chip and so on. Since the beginning of human genome project, gene scanning and genotyping techniques have developed rapidly, which are widely used in pathogenic gene screening, genetic linkage and association analysis of disease genes, early diagnosis for diseases like cancers, individual identification in forensic uses and so on. An important means of genotyping is to analyze the molecular weight of gene fragment using a DNA sequencer or genetic analyzer. When this kind of equipment is used to analyze molecular weight, a molecular weight standard is needed to calculate the molecular weight of sample, which is molecular weight standard , also known as internal lane standard.

A commonly used molecular weight standard is LIZ-500, which comprises 15 double-stranded DNA fragments labeled with LIZ fluorescence (red), the molecular weight of which are 35bp, 50bp, 75bp, 100bp, 139bp, 150bp, 160bp, 200bp, 250bp, 300bp, 340bp, 350bp, 400bp, 450bp, 490bp and 500bp, respectively, and the differences in molecular weight between the DNA fragments are greater than or equal to 10bp. These internal standard fragments are obtained by digesting the plasmid and then ligating fluorescence to the oligonucleotide. In STR analysis, due to the reason that buffer in the buffer bath, water in the water bath, capillary tube, POP and formamide of the Genetic Analyzer may be expired or unqualified, and a large quantity of amplified fragments are generated, when the size of the amplified fragments are relative large and similar in size, and the labeled primers have the same color, the conventional molecular weight standard used in DNA STR analysis cannot ensure the single-base resolution, or ensure the accuracy of genotyping when buffer, water, capillary tube, POP or formamide is expired or unqualified.

### SUMMARY

Aiming at the deficiencies of conventional molecular weight standard in use, the present disclosure provides a molecular weight standard and use thereof, which can identify single base clearly and ensure the accuracy of genotyping, thereby facilitating the operators to use.

In order to achieve the goal of the present disclosure, the present disclosure takes the following technical solutions.

A molecular weight standard, comprising n nucleotide fragments that have different molecular weight and identifiable labels, which contains at least p pairs of nucleotide fragments differing by one base; in the nucleotide fragment pair that have one base difference, the number of base in one fragment is m and the number of base in the other fragment is m+1 or m-1.

In some embodiments, when n is an even number, p is from 1 to n/2. That is, at least 1 to n/2 pairs of nucleotide fragments differing by one base are included. For example, if n is 10, p can be from 1 to 10/2. That is, 1 pair, 2 pairs, 3 pairs, 4 pairs or 5 pairs of nucleotide fragments differing by one base may be included.

In some embodiments, when n is an odd number, p is from 1 to (n-1)/2. That is, at least 1 to (n-1)/2 pair of nucleotide fragments differing by one base is included. For example, if n is 11, p can be from 1 to (11-1)/2. That is, 1 pair, 2 pairs, 3 pairs, 4 pairs or 5 pairs of nucleotide fragments differing by one base may be included.

In some preferred embodiments, the molecular weight standard of the present disclosure has a p from 2 to 6. That is, the molecular weight standard of the present disclosure comprises 2 to 6 pairs of nucleotide fragments differing by one base.

In some embodiments, the number of base of molecular weight standard in the present disclosure, represented by m, is from 30 to 5000.

In some embodiments, m is from 30 to 1000.

In some preferred examples, m is from 30 to 600.

In some embodiments, the identifiable label of the molecular weight standard of the present disclosure is fluorescent label, which has an emission spectrum ranging from 400nm to 1000nm.

Preferably, the fluorescent labels include, but are not limited to, the following fluorescein: LIZ, FAM, HEX, JOE, TMR, TET, VIC, NH635 and Cy5.

The present disclosure also provides use of the molecular weight standard in fragment analysis.

Preferably, the fragment analysis includes, but is not limited to, STR analysis, sequencing, large fragment analysis and genome analysis.

The present disclosure also provides a fragment analysis kit comprising the molecular weight standard of the present disclosure.

The molecular weight standard prepared in the present disclosure is a DNA molecule with a fluorescent label. The general operating method is as follows: deionized formamide is added to the molecular weight standard before being heated at 95°C for 3 to 5min for denaturation, and then the mixture is quickly placed on ice and cooled for 3min.

According to the technical solutions above, the present disclosure provides a molecular weight standard, comprising n nucleotide fragments having different molecular weight and identifiable label; wherein at least p pairs of nucleotide fragments differing by one base are included; one fragment from the pair of nucleotide fragments differing by one base has a base number of m, the other has a base number of m+1 or m-1. Comparing with the existing commercial molecular weight standard, that of the present disclosure is capable to discern single base, which can efficiently distinguish PCR product fragments differing by one base, and ensure correct genotyping, to facilitate the operators to interpret, thereby being wildly used in STR analysis, sequencing, large fragment analysis and genome analysis.

### BRIEF DESCRIPTION OF DRAWINGS

In order to describe the technical solutions in the examples of the present disclosure or the prior art more clearly, the accompanying drawings used in description of the embodiments or the prior art will be illustrated briefly.
Figure 1 shows the STR analysis results of Example 2. Figure 1a shows the Fam labeled product fragment; Figure 1b shows the Hex labeled product fragment, Figure 1c shows the Tamra labeled product fragment; Figure Id shows the molecular weight standard of present disclosure; the abscissa is the base number and the ordinate is the signal intensity.
Figure 2 shows the co-electrophoresis analysis results of conventional molecular weight standard and gene sample in Example 2. Figure 2a shows the Fam labeled product fragment: A stands for the Fam labeled product fragment, locating within Bin, which is represented by the dark vertical stripes on the background; B stands for the Fam labeled product fragment, which is located outside Bin. Figure 2b shows the conventional molecular weight standard; the abscissa is the number of base and the ordinate is the signal intensity.
Figure 3 shows the co-electrophoresis analysis results of molecular weight standard of the present disclosure and gene sample in Example 2. Figure 3a shows the Fam labeled product fragment: A stands for the Fam labeled product fragment locating within Bin; B is the Fam labeled product fragment, which is located outside Bin. Figure 3b shows the molecular weight standard of the present disclosure; the abscissa is the number of base and the ordinate is the signal intensity.
Figure 4 shows the capillary electrophoresis spectrogram of the molecular weight standard in Example 2 of the present disclosure, wherein the abscissa is the number of base and the ordinate is the signal intensity.
Figure 5 is an enlarged view of 75bp and 76bp signal in Figure 4, wherein the abscissa is the number of base and the ordinate is the signal intensity.
Figure 6 is an enlarged view of 500bp and 501bp signal in Figure 4, therein, the abscissa is the number of base and the ordinate is the signal intensity.

### DETAILED DESCRIPTION

The technical solutions in the embodiments of the present disclosure will be described clearly and completely herein in conjunction with the examples of the present disclosure. Apparently, the described examples are only a part of the examples of the present disclosure, rather than all examples. Based on the examples in the present disclosure, all of other examples, made by those skilled in the art without any creative efforts, fall into the protection scope of the present disclosure.

### Example 1: Preparation method for the new-type molecular weight standard of the present disclosure

The molecular weight standard can be prepared by Polymerase Chain Reaction (PCR) or enzyme digestion. The two methods will be illustrated separately in detail below, so that those skilled in the art can carry them out without creative efforts.

### 1. PCR method

### 1.1 Design and synthesis of upstream primer and downstream primer

Primer Express was used to analyze a widely used exogenous DNA template. A primer was designed at upstream (5') and labeled with NH635 at the 5' terminal. Upstream primer 5'-GCCCGTCGAGAATACTGGCAATTTCACCTGC-3' has an annealing temperature of 58°C. The upstream primer was set as the starting point, and the ending point of the downstream primer was set at the desired distance of the downstream. The length and sequence of the downstream primer should ensure the downstream primer to possess the following features: relative high specificity, similar annealing temperature with the upstream primer, GC content of 20% to 40%, no complex secondary structure, and no dimers formed with the upstream primer.

### 1.2 PCR Amplification

After the synthesis of upstream primer and downstream primer, the primers were diluted to 10pM with TE. The condition for the amplification was explored and the products were amplified under the selected condition and analyzed. The amplification was performed on AB9700 Thermocycler.

The final reaction system and reaction condition were shown in Table 1 as follows:

**Table 1 Reaction system**

| Component | Final Concentration | Added Volume (µl) |
|---|---|---|
| HotStart Taq(5U/µl) | 2u/Reaction | 0.4 |
| 100mM dNTPs | 0.2mm/Each | 0.8 |
| Exogenous DNA(5 ng/µl) | 1×1 0ng/Reaction | 2 |
| 10×Commercial PCR Buffer | 1× | 10 |
| 10×Primer (4µM) | 200nM | 5 |
| ddH₂O | | 81.8 |
| Total | | 100 |

The reaction conditions were: 95°C 11min; 30 cycles: 94°C 30sec, 59°C 60sec, 68°C 60sec; 60 °C 60min.

### 1.3 Adjust the amount of product to balance the peak of each band

Target fragments amplified by the polymerase chain reaction were tested by capillary electrophoresis to make sure whether the size is correct or not. Thereafter, the area of each peak was analyzed. Target fragments were mixed and equilibrated until the differences between the heights of the peaks of all the target fragments were less than or equal to 10%.

The content of each band was adjusted to reach equal molar proportions and show relatively uniform peaks. Analysis was carried out by ABI3130 Genetic Analyzer as follows:
1) 0.5µL of the test sample and 9.5µL of formamide were mixed and denatured at 95°C for 3min; then the mixture was quickly placed on ice.
2) The mixture was detected using ABI3130 genetic analyzer with parameters set as follows: injection voltage of 1kV; injection duration of 15s; electrophoresis voltage of 15kV; electrophoresis duration of 1400s.
3) Each peak of the fragments has a height over 500RFU.

### Example 2: STR Analysis

The molecular weight standard prepared according to the methods described in Example 1 comprised fragments of 75, 76, 80, 100, 140, 160, 161, 175, 180, 200, 225, 226, 250, 275, 15 300, 330, 331, 360, 390, 445, 500 and 501bp long.

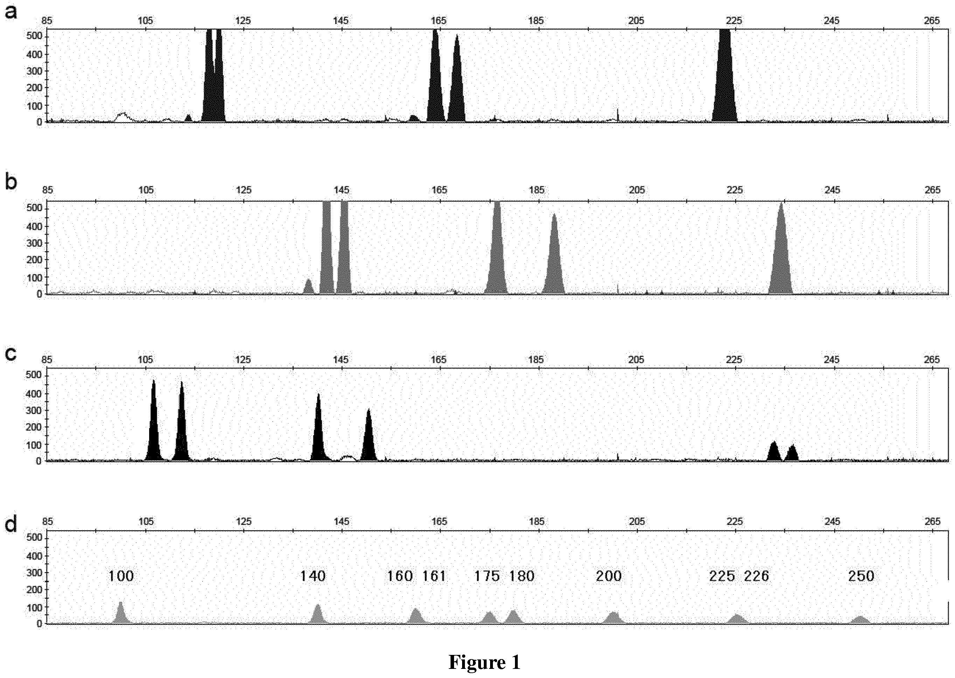

STR analysis was carried out using the molecular weight standard of the present disclosure under the condition of expired or unqualified Buffer, water, capillary tube, POP glue or formamide, and the results were shown in Figure 1.

Figure 1 showed that the molecular weight standard of the present disclosure can distinguish the single base difference between 160bp and 161bp, 225bp and 226bp, ensuring single base resolution; however, LIZ-500 molecular weight standard cannot distinguish 160bp and 161bp, or 225bp and 226bp, failing to distinguish single base or ensure correct genotyping result.

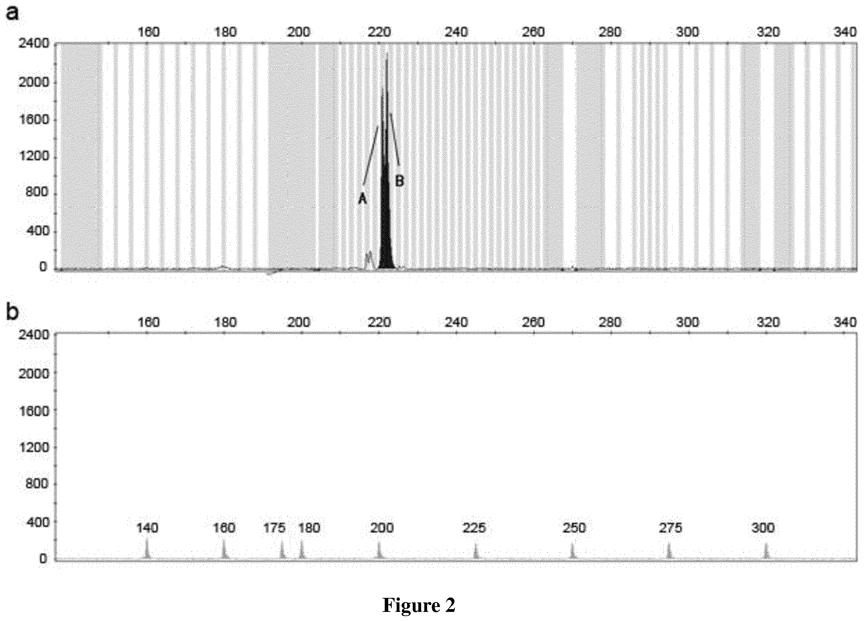

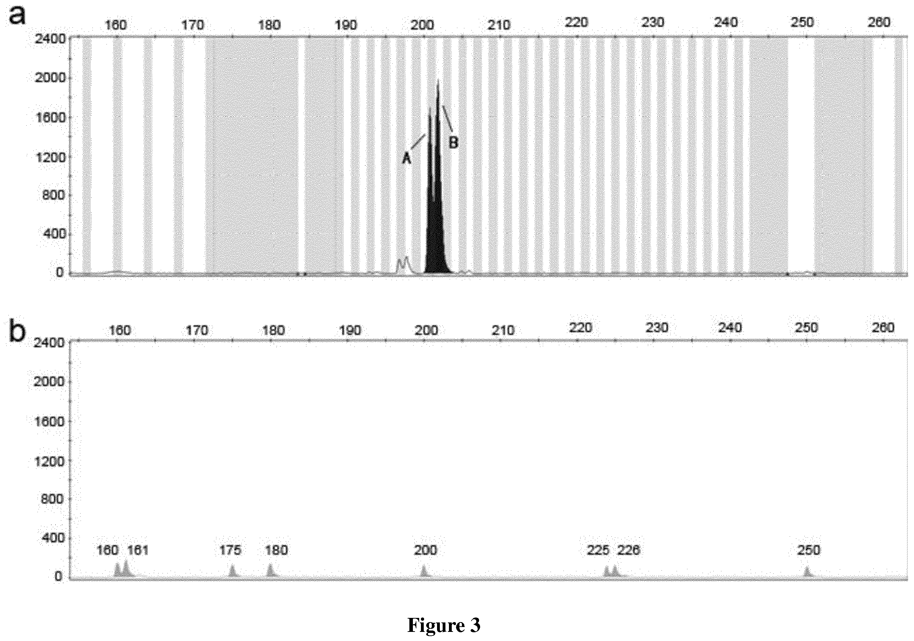

The molecular weight standard of the present disclosure and Liz-500 molecular weight standard were used respectively to analyze two adjacent fragments that were not located within Bin, the results were shown in Figure 2 and Figure 3.

Figure 2 and Figure 3 showed that molecular weight standard Liz-500 cannot determine whether the fragments not located within Bin were product peaks, while the molecular weight standard of the present disclosure can determine whether the fragments not located within the Bin were correct product peaks.

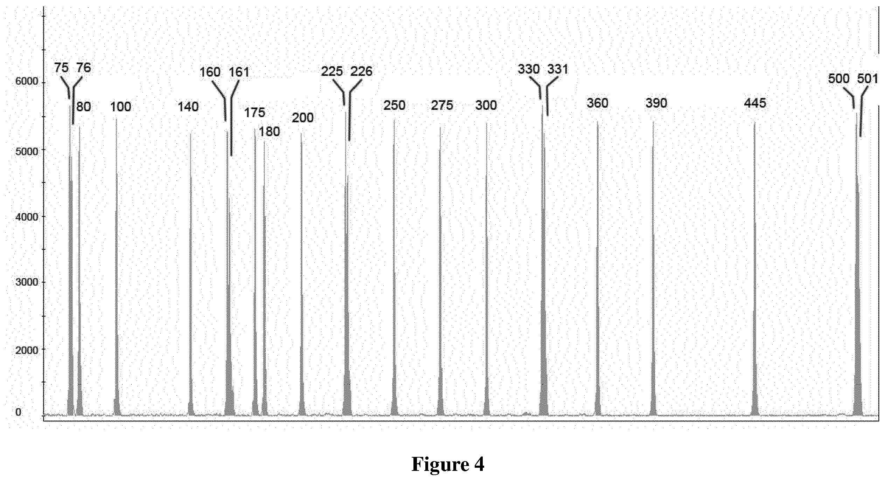

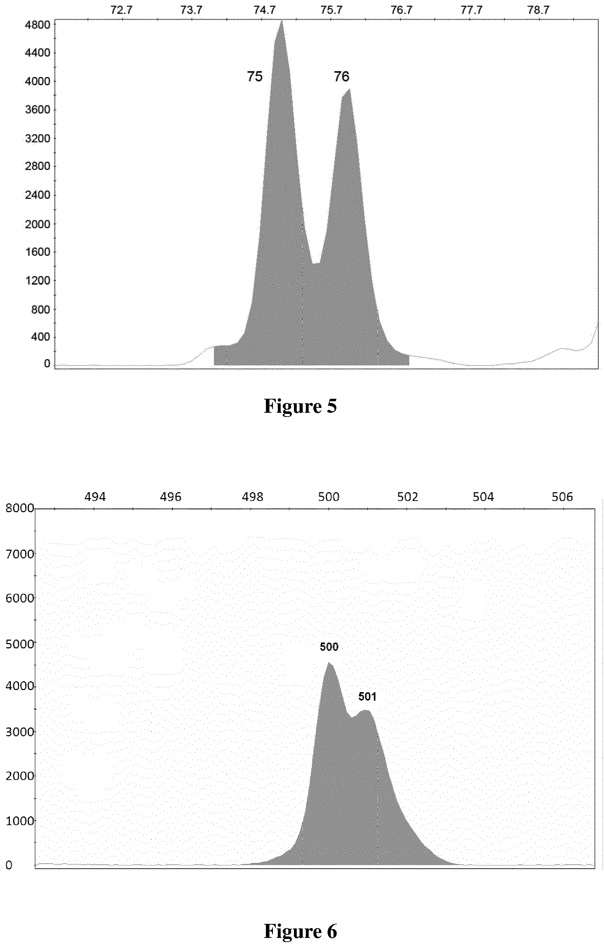

The molecular weight standard of the present disclosure was further analyzed on sequencer and the results were shown in Figures 4 to 6.

Figures 4 to 6 showed that the molecular weight standard of the present disclosure is distinguishable between 75bp and 76bp, 160bp and 161bp, 225bp and 226bp, 330bp and 331bp, 500bp and 501bp on a sequencer, indicating that in the analysis area the molecular weight standard of the present disclosure has the ability to identify single base difference, thereby efficiently distinguishing the PCR products differing by one base to facilitate the operators to interpret.

The above is only the preferred examples of the present invention, and it should be noted that those skilled in the art can make improvements and modifications without departing from the principle of the present invention, and these improvements and modifications should be regarded within the protection scope of the present invention.

## Claims

1. A molecular weight standard, consisting of n nucleotide fragments having different molecular weight and identifiable label, which comprises at least p pairs of nucleotide fragments differing by one base; wherein the base number of one fragment of the nucleotide fragment pair differing by one base is m, and the base number of the other fragment is m+1 or m-1.

2. The molecular weight standard according to claim 1, wherein,
when n is an even number, p is from 1 to n/2;
when n is an odd number, p is from 1 to (n-1)/2.

3. The molecular weight standard according to claim 2, wherein p is from 2 to 6.

4. The molecular weight standard according to any of claims 1 to 3, wherein 30<m<5000, preferably 30<m<1000.

5. The molecular weight standard according to any of claims 1 to 3, wherein 30<m<600.

6. The molecular weight standard according to any of claims 1 to 5, wherein the identifiable label is fluorescent label which has an emission spectrum from 400nm to 1000nm.

7. The molecular weight standard according to claim 6, wherein the fluorescence label is selected from the group consisting of LIZ, FAM, HEX, JOE, TMR, TET, VIC, NH635 and Cy5.

8. Use of the molecular weight standard according to any of claims 1 to 7 in fragment analysis.

9. The use according to claim 8, wherein the fragment analysis is selected from the group consisting of STR analysis, sequencing, large fragment analysis and genome analysis.

10. A fragment analysis kit, comprising the molecular weight standard according to any of claims 1 to 9.
